# EUROPEAN PATENT SPECIFICATION

(11) **EP 4 007 626 B1**
(45) Date of publication and mention of the grant of the patent: **13.09.2023**
(21) Application number: 20742299.9
(22) Date of filing: 23.07.2020
(51) Int. Cl.: A61M 11/00, A61M 15/00

(54) **BUCCAL ADMINISTRATION OF AEROSOL**
BUKKALE VERABREICHUNG VON AEROSOL
ADMINISTRATION BUCCALE D'AÉROSOL

(30) Priority: 02.08.2019 EP 19189887
(43) Date of publication of application: 08.06.2022
(73) Proprietor: Stamford Devices Limited, Dangan, Galway (IE)
(72) Inventor: TELFER, Colin, Dangan, Galway (IE); POWER, Patrick, Oranmore, Galway (IE)
(74) Representative: Weldon O'Brien Ltd.
(86) International application number: PCT/EP2020/070890
(87) International publication number: WO 2021/023522

(56) References cited:
- EP-A1- 1 439 875
- WO-A1-2004/041336
- WO-A1-2009/013501
- WO-A1-2009/111612
- WO-A1-2010/131188
- WO-A1-2018/216019
- WO-A2-2006/102345
- US-A1- 2002 129 813
- US-A1- 2005 011 514
- US-A1- 2007 267 010
- US-A1- 2014 116 426

## Description

### Introduction

This invention relates to medical aerosol delivery and buccal administration using an aerosol generator.

WO2019/079461 (Pneuma Respiratory INC) describes devices for nasal delivery for topical treatment of local diseases in the nose and paranasal sinuses, such as allergic and non-allergic rhinitis and sinusitis.

WO2018/216019 (Solomon) describes delivering of a liquid aerosol into an oral cavity of a user by suction from a mouth of the user, said apparatus using a mouthpiece.

WO2014046993 (Edwards) describes a universal flow diverter that can be connected to the mouthpiece of an aerosol delivery device.

US2011/0168170 (Patton) describes preservative free insulin formulations and systems and methods for aerosolizing.

WO02074372 (Papania) describes aerosol delivery systems with an insulated receptacle connected to a body to hold a vial of an agent to be delivered to a patient.

WO2017192767 (Germinario) describes a droplet delivery device for precise and repeatable dosages to a subject for pulmonary use.

WO2010/131188 (Philips) describes a drug delivery apparatus. WO2009/111612 (Novartis) describes an aerosolization device with a fluid pathway for liquid to impinge upon an aerosol generator. WO2009/013501 (Respironics) describes a drug delivery apparatus with a mouthpiece portion and an aerosol generator, wherein the aerosol generation is initiated in response to a sensor (e.g. flow rate sensor) detecting the breathing pattern including inhalation and exhalation.

US2014/116426 (Mullinger B) describes an inhalation device comprising a base unit, a mouthpiece, and an aerosol head, designed to allow oral inhalation of the nebulised aerosol, i.e. inhalation via the mouth, to deliver aerosols to the lungs, ideally reaching even the smallest branches of the peripheral lungs, such as bronchioles and alveoli. An abrupt decrease of the cross-sectional area within the injection zone (where the aerosol droplets are introduced from the aerosol generator into the air channel) is disclosed to accelerate the air flow in the narrowed, or constricted, region and cause turbulences. While these turbulences ensure sufficient mixing of the nascent aerosol droplets with air, the accelerated air flow reduces the density of the nascent aerosol by increasing the distance between the individual droplets in the direction of the air flow and thus avoids undesirable coalescence.

US2002/129813 (Litherland) describes a method for aerosolizing a liquid which comprises providing an aerosol generator having a plate with a plurality of apertures and a piezoelectric element to vibrate the plate. US2005/011514 (Power) describes a nebuliser to deliver a medicament that includes a housing having a reservoir for the medicament, and an aerosol generator that can be supplied with the medicament from the reservoir, and that includes a vibratory thin shell member with a plurality of apertures having a size range of about 1 to about 6 microns.

EP1439875 (Aerogen) describes an apparatus for delivery of a medicament to a respiratory system which comprises a reservoir, an aerosol generator for aerosolising liquid medicament and a connector for entraining the aerosolised medicament from the aerosol generator with a gas.

WO2018/216019 (Soloman) describes an apparatus for delivering a liquid aerosol to oral cavity surfaces. WO2006/102345 (Aerogen) and US2007/267010 (Fink et al) describe methods of treating a patient with a pulmonary disease, where the method includes delivering a dose of aerosolized medicament intermittently to a ventilator circuit coupled to the respiratory system of the patient, wherein a controller is operable to allow for a choice of modes of operation, for example, a mode in which aerosolization begins once a certain breath characteristic is detected, such as after the inhalation phase of the cycle before exhalation has begun.

WO2004/041336 (PARI) describes an inhalation therapy device with an aerosol generator and a mixing chamber.

Existing technologies generate aerosol sprays with a broad distribution of droplet sizes, with a percentage within the respirable range. Devices such as those described in EP2273933 use sensors to provide feedback to a controller so that aerosol is provided in the inhaled breath, the aerosol output is adjusted to the inspiratory flow.

The invention is directed towards providing an apparatus for improved buccal delivery of aerosol.

### Statements of Invention

The invention provides a nebulizer device as set out in claim 1, and optional features are set out in the dependent claims.

We describe a nebulizer device comprising an aerosol generator mounted in a housing, a controller, and a mouthpiece, the device being adapted for delivery of an active agent in an aerosol into the buccal cavity.

Preferably, the aerosol generator is adapted to provide droplets for which a majority fraction has a size greater than 10µm. Preferably, the aperture plate has apertures with an exit diameter in excess of 6.8 µm. Preferably, the majority fraction is 75%, and preferably 90%.

Preferably, the features comprise surface formations such as dimples.

Preferably, the controller is configured for operation of the aerosol generator responsive to nasal exhalation. Preferably, the detector comprises one or more of temperature, pressure, flow or auditory sensors.

Preferably, the controller is configured to control a method of operation including the steps of:
providing a liquid containing an active substance;
aerosolising the liquid to provide an aerosol comprising droplets having an average mean diameter of greater than 10µm;
starting aerosol generation at or after the end of breath inhalation;
delivering the aerosol into the buccal cavity; and
stopping aerosol generation at or before the start of breath inhalation.

Preferably, the controller is adapted to vary drive voltage for the aperture plate in order to achieve a desired droplet size. Preferably, the device comprises an aperture plate with aerosol-forming apertures, and the aperture plate comprises a reservoir layer with cavities forming liquid supply openings for the aerosol-forming apertures.

The device comprises a narrowed portion of the mouthpiece or other channel leading to the mouthpiece, to encourage droplet collisions and thereby achieve large droplet sizes. Preferably, the aperture plate has a number of aerosol-forming apertures in excess of 200, and preferably in excess of 1000.

We also describe a method, not being part of the claimed invention, for administration of an active agent into the buccal cavity, the method being performed by a device of any example described herein, and comprising the steps of:
providing a liquid containing an active substance;
aerosolising the liquid to provide an aerosol comprising droplets having an average mean diameter of greater than 10µm;
starting aerosol generation at or after the end of breath inhalation;
delivering the aerosol into the buccal cavity; and
stopping aerosol generation at or before the start of breath inhalation.

Preferably, the method comprises detecting the end of breath inhalation and starting aerosol generation based on the detection of the end of breath inhalation. Preferably, the method comprises detecting the start of breath inhalation and stopping aerosol generation based on the detection of the start of breath inhalation. Preferably, the method comprises monitoring a breathing profile of a patient and controlling the aerosol generation such that aerosol is generated only when the patient is not inhaling.

Preferably, the method comprises monitoring a breathing profile of a patient and stopping generation of aerosol in the event of irregular inhalation. Preferably, the method comprises detecting nasal exhalation and starting aerosol generation based on the detection of the start of nasal exhalation. Preferably, the method comprises detecting nasal exhalation and stopping aerosol generation based on the detection of the end of nasal exhalation.

### Brief Description of the Drawings

The invention will be more clearly understood from the following description thereof, given by way of example only, in which:

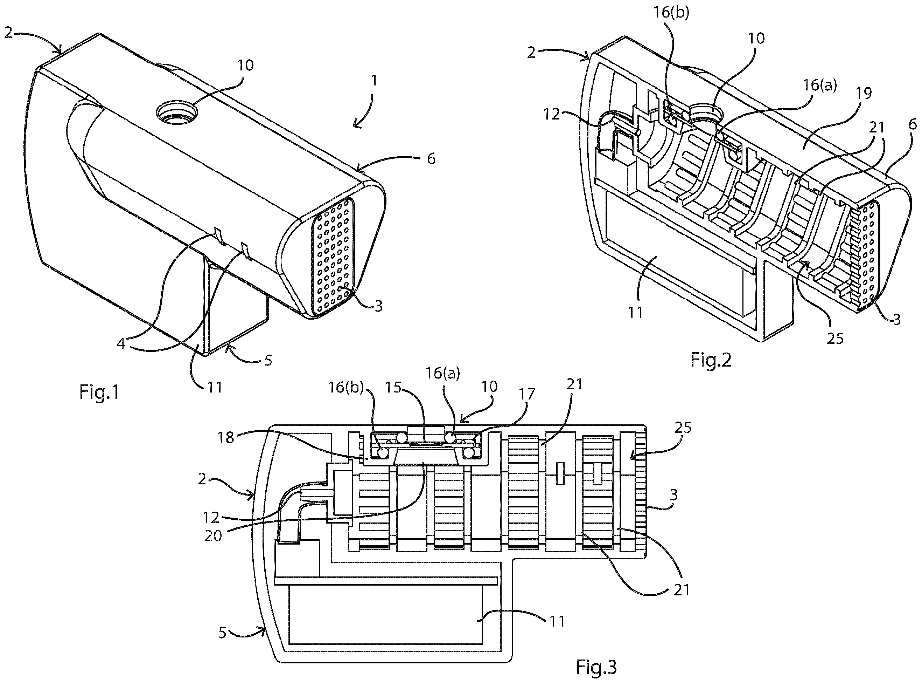

Fig. 1 is a perspective view of a nebulizer of the invention; and
Fig. 2 is a cut-away perspective view showing particularly a delivery conduit for outlet of aerosol, and Fig. 3 is a cut away side view also showing these features.

### Detailed Description

### Device Physical Aspects

Referring to the drawings, a nebulizer 1 has a hand-held housing 2 forming a handle 5 and a mouthpiece 6 protruding from the handle 5. The handle 5 houses a controller 11 with a battery power source. The housing 2 at the distal end of the mouthpiece 6 also supports a mesh 3 for outlet of aerosol into a user's mouth. Inlet air flow is provided by inlet vents 4 on each lateral side of the housing 2 mouthpiece 6.

Within the housing 2 there is an aerosol generator 10, in this case comprising an aperture plate 15 mounted on a washer 17 to which is also mounted a piezoelectric actuator powered and driven by the controller 11 for causing vibration of the aperture plate 15 at a frequency in the region of 128kHz in one example. The washer 17 is supported by a pair of opposed O-rings 16(a) and 16(b) in a manner akin to that described in our published PCT specification WO2012044220, but with power being provided by leads and solder joints.

The support washer 17 engages against the lower O-ring 16(b) which is housed in an annular groove 18 of the housing 2. The support washer 17 also engages against the upper O-ring 16(a) under an upper wall 19 of the housing 2. The washer 17 is sandwiched between the upper and lower O rings 16 as a subassembly which is in turn sandwiched between the upper housing wall 19 and the lower retaining housing annular groove 18. The arrangement of the lower retaining groove 18 provides uniform support around the washer 17 upper, counter-balanced by the support forces applied via the upper O-ring 16(a). This helps to achieve consistent operation of the aerosol generator 10, with reduced risk of fatigue in the seal between the plate 15 and the support washer 17, and a predictable plate vibration response to the applied electrical drive.

The overall configuration is that the housing 2 forms an elongate mouthpiece 6 which provides a flow path 25 for flow of aerosol to and through the mesh 3. The order, from a proximal end (furthest from the user in use) is:
a pressure sensor 12 at the proximal end, which is closed;
aerosol generator 10, in an uppermost side of the flow path 25 in use;
air inlet vents 4 at the sides of the main volume of the flow path 25; and the mesh 3 for outlet of aerosol into the mouth.

The housing 2 comprises features for enhancing turbulent flow of air with entrained aerosol.

The features comprise surface formations, preferably such as dimples or ridges.

The device comprises one or more narrowed portion of the mouthpiece 6, to encourage droplet collisions and thereby achieve large droplet sizes. Also, as described in more detail below, the sensor 12 feedback to the controller is used by the controller to cause aerosolization to commence with patient exhalation. Hence, the controller is configured for operation of the aerosol generator responsive to nasal exhalation.

In various other examples a sensor may comprises one or more of temperature, pressure, flow or auditory sensors.

The pressure sensor 12 is mounted in the housing 2, in a dead space upstream and proximally of the aerosol generator 10 at the proximal end of the flow conduit 25. The aerosol generator 10 is actuated when inhalation has ended or is about to end. During exhalation there is increased pressure in the flow path 25, arising from patient's breath. The controller 11 is programmed to recognize start of exhalation upon an increase of pressure sensed by the sensor 12 above a threshold.

In other examples, for the turbulent flow there may be a pattern of cylindrical bumps along with ribs which narrow the diameter. Turbulence may also be achieved by erratically shaped bumps and pits throughout the flow path.

### Controller Operation

The controller 11 uses the pressure sensor 12 to track the patient's breathing pattern. The controller 11 then uses the tracked data to predict when subsequent inhalations will begin. This allows the controller 11 to direct the piezoelectric actuator to vibrate the aperture plate 15 to begin aerosolizing the liquid within a predetermined time period (e.g., +/-0.5 seconds) before or after predicted start of exhalation. While the aerosol delivery controller 11 is able to predict the breath cycle to produce aerosol for the patient, it is also configured to recognize spontaneous/irregular breathing not part of the normal pattern using the breath sensor 12. Once a spontaneous breath exhalation is recognized, the controller 11 may immediately operate the aerosol generator 10 for delivery of aerosol to the patient.

The aperture plate is vibrated at ultrasonic frequencies to produce liquid droplets. Some specific, non-limiting examples of technologies for producing fine liquid droplets is by supplying liquid to an aperture plate having a plurality of tapered apertures extending between a first surface and a second surface thereof and vibrating the aperture plate to eject liquid droplets through the apertures. Such technologies are described generally in U.S. Pat. Nos. 5,164,740; 5,938,117; 5,586,550; 5,758,637; 6,014,970, 6,085,740, and US2005/021766A.

However, it should be appreciated that the present invention is not limited for use only with such devices.

Various methods of controlling the operation of such nebulisers or aerosol generators are described in US Pat Nos. 6,540,154, 6,845,770, 5,938,117 and 6,546,927.

In one example, the controller 11 includes a stored value that is an estimate of a delivery time period to essentially fill the mouthpiece flow conduit 25 within the housing 2. The controller may include a memory for storing the operation time period, and it may calculate and store an initialization time period. The controller 11 may calculate the operation time period by subtracting the stored value from the initialization time period. In this manner, each time the user exhales, aerosol production begins when the threshold flow rate is achieved and ends after the operation time period that has already been calculated and stored in the controller.

The controller 11 may drive the piezo actuator in a manner in which end of a liquid dose on the aperture plate is determined by virtue of monitoring the drive parameters. This may be done in a manner as described in WO2015/010809.

In this case, the controller 11 measures an electrical drive parameter at each of a plurality of measuring points, each measuring point having a drive frequency; and based on the values of the parameter at the measuring points makes a determination of optimum drive frequency and also an end-of-dose prediction. The controller performs a short scan at regular sub-second intervals at which drive current is measured at two measuring points with different drive frequencies. According to drive parameter measurements at these points the controller determines if a full scan sweeping across a larger number of measuring points should be performed. The full scan provides the optimum drive frequency for the device and also an end of dose indication.

However, the aerosol generator and its controller may alternatively be of any suitable type known in the art.

The aerosol generator 10 has an outlet 20 in the flow path 25 terminating in the mesh 3. The flow path is formed by internal surfaces of the mouthpiece part of the housing 2, which in this case have ribs 21 extending around and into the path in a direction perpendicular to the aerosol flow direction. Physical features of any other shape may be used, provided they help to create turbulence so that droplets coalesce in the flow path 25. The features narrow the flow path, and ribs as illustrated are particularly effective.

The device 1 performs delivery of aerosol to the mouth with any desired active agent.

There is control of delivery during a particular portion of the breath cycle, particularly during exhalation.

In one example, the nebulizer 1 performs a method for administration of an active agent into the buccal cavity comprising the steps of:
providing a liquid containing an active substance on the aperture plate 15;
aerosolising the liquid to provide an aerosol comprising droplets having an average mean diameter of greater than 10µm;
starting aerosol generation at or after the end of breath inhalation;
delivering the aerosol into the buccal cavity; and
stopping aerosol generation at or before the start of breath inhalation.

In various examples, nebulizers of various examples perform one or more of:
detecting the end of breath inhalation and starting aerosol generation based on the detection of the end of breath inhalation,
detecting the start of breath inhalation and stopping aerosol generation based on the detection of the start of breath inhalation,
monitoring a breathing profile of a patient and controlling the aerosol generation such that aerosol is generated only when the patient is not inhaling,
monitoring a breathing profile of a patient and stopping generation of aerosol in the event of irregular inhalation,
detecting nasal exhalation and starting aerosol generation based on the detection of the start of nasal exhalation,
detecting nasal exhalation and stopping aerosol generation based on the detection of the end of nasal exhalation,

The device may comprise directing means for directing an exhalation flow towards the aerosol generator for entrainment of aerosol.

As described above the mouthpiece and/or the housing comprise features for enhancing turbulent flow of air with entrained aerosol. These features comprise surface formations, preferably such as dimples, and in the example illustrated they are primarily ribs around the flow path.

The operation of the aerosol generator 10 may be responsive to the end of breath inhalation and/or the start of breath inhalation. In one case the operation of the aerosol generator is responsive to nasal exhalation. The device comprises a detector for detecting inhalation and/or exhalation. In some cases, the detector comprises one or more of temperature, pressure, flow or auditory sensor.

### Large Aerosol Droplet Size

The aerosol droplets are in a range which is unsuited for respiration. In one example a majority of the droplets has a size greater than 10µm . The majority is preferably 60%, more preferably 75%, and more preferably 90%. This is achieved by the aperture size of the aperture plate 15, in which the exit diameter of the aerosol-forming apertures is greater than 5 µm and preferably greater than 6.8 µm. In one example this is achieved in an electroforming manufacturing process.

In other examples the large droplet size is achieved at least in part by control of aperture plate drive voltage.

It is envisaged that the aperture plate may have a reservoir layer with cavities forming liquid supply openings for the aerosol-forming apertures, especially if the droplet size is towards the lower end of above range. This would assist in achieving the flow rates as set out below, and might assist nebulization of a wider range of formulations.

The nebulizer provides the desired large droplet size at least in part by having a narrowed portion of the mouthpiece (especially the components 6, 26, 21, 3) or other channel, to encourage more droplet collisions.

In general, it is preferred that the aperture plate has a number of aerosol-forming apertures in excess of 200, and preferably in excess of 1000.

### Flow Rates

Also, the flow rates are much greater than is typical. Preferably, the flow rates are in excess of 1ml/min and below 5 ml/min when flow is occurring, and more preferably in the range of 2ml/min and 4ml/min, and more preferably approximately 3ml/min. Of course, averaged including the non-delivery periods (during inhalation) the average flow rate will be correspondingly lower.

An excessive flow rate might cause rain-out and the patient swallowing rather than the desired absorption occurring.

As noted above, the nebulizer 1 incorporates a breath actuation system such that the aerosol generation is triggered at the end of inhalation and stopped upon inhalation start (i.e. whenever the patient is not inhaling) ensuring that the aerosol is delivered to the oral cavity. In various examples, the breath actuation system may operate from feedback from a pressure, flow, auditory and/or temperature sensor which will map the patient's breathing profile. In the event of an unexpected inhalation occurring, aerosolization is immediately ceased. Breath actuation *per se* is well known in the field and the device may employ any effective breath sensing and actuation system.

The trigger may be configured to be dependent on nasal exhalation (a person cannot exhale through their nose and inhale through their mouth simultaneously). A nasal exhalation trigger may utilise a pressure, flow, auditory and/or temperature sensor.

It will be appreciated that the aerosol exit port/mouthpiece features ensure turbulent flow and thereby maximise rainout within the oral cavity.

To assist in driving aerosol through the flow path from the aerosol generator to the oral cavity, a percentage of exhalation may be directed down flow paths on the mouthpiece to a location behind the aerosol generator. This will carry aerosol into the buccal cavity.

This invention broadly mitigates the inhalation risks present with existing technologies in that it delivers a finely controlled dose to the buccal cavity which prevents inhalation of aerosol.

The devices of the various examples provide very effective delivery of agents such as therapeutics solely for buccal administration, with delivery efficiency to the oral mucosa, with little risk of aerosol inhalation beyond the oral cavity.

The invention is not limited to the embodiment hereinbefore described, which may be varied in detail within the scope of the claims.

## Claims

1. A nebulizer device (1) comprising an aerosol generator (10) mounted in a housing (2), a controller (11), and a mouthpiece (6, 3), the device being adapted for delivery of an active agent in an aerosol into the buccal cavity,
wherein:
the aerosol generator comprises an aperture plate (15) and a vibration actuator (11, 17) for vibrating the aperture plate at a frequency for aerosolization of a liquid, wherein the aperture plate has apertures with an exit diameter in excess of 5.0 µm;
the device comprises a directing means (3, 6) for directing an exhalation flow towards the aerosol generator (10) for entrainment of aerosol;
the mouthpiece comprises features (21) for enhancing turbulent flow of air with entrained aerosol, said features comprising surface formations (21) in internal surfaces of a flow conduit (6, 25) providing a flow path in the mouthpiece, and narrowing said flow path;
the device comprises a detector (12) for detecting inhalation and/or exhalation; and
the controller (11) is configured to operate such that operation of the aerosol generator (10) is responsive to the end of breath inhalation.

2. A device as claimed in claim 1, wherein the aerosol generator (10) is adapted to provide droplets for which a majority fraction has a size greater than 10µm.

3. A device as claimed in claims 1 or 2, wherein the aperture plate has apertures with an exit diameter in excess of 6.8 µm.

4. A device as claimed in claims 2 or 3, wherein the majority fraction is 75%, and preferably 90%.

5. A device as claimed in any preceding claim, wherein the features comprise dimples or ridges (21).

6. A device as claimed in any preceding claim, wherein the controller is configured for operation of the aerosol generator (10) responsive to nasal exhalation.

7. A device as claimed in any preceding claim, wherein the detector comprises one or more of temperature, pressure, flow or auditory sensors.

8. A device as claimed in claim 7, wherein the sensor comprises a pressure sensor (12), and the pressure sensor (12) is mounted upstream of the aerosol generator (10) at or adjacent a closed end of a chamber (25) proximally of an outlet (3) of the mouthpiece.

9. A device as claimed in claim 8, wherein the mouthpiece (6) comprises, in order from proximal to distal towards the user's mouth in use: a flow or pressure sensor (12), the aerosol generator (10), a volume (25) formed by a housing (2, 6) wall with features (21) which narrow the flow path, and an outlet mesh (3).

10. A device as claimed in any preceding claim, wherein the controller is configured to control the operation of the device including the steps of:
aerosolising a liquid containing an active substance to provide an aerosol comprising droplets having an average mean diameter of greater than 10µm;
starting aerosol generation at or after the end of breath inhalation;
delivering the aerosol into the buccal cavity; and
stopping aerosol generation at or before the start of breath inhalation.

11. A device as claimed in any preceding claim, wherein the controller (11) is adapted to vary drive voltage for the aperture plate (15) in order to achieve a desired droplet size.

12. A device as claimed in any preceding claim, wherein the aerosol generator comprises an aperture plate with aerosol-forming apertures, and the aperture plate comprises a reservoir layer with cavities forming liquid supply openings for the aerosol-forming apertures.

13. A device as claimed in any preceding claim, wherein the aerosol generator comprises an aperture plate which has a number of aerosol-forming apertures in excess of 200, and preferably in excess of 1000.

14. A device as claimed in any preceding claim, wherein the controller (11) and the aerosol generator (10) are configured to provide flow rates of aerosol in excess of 1mL/min and below 5 mL/min.

15. A device of claim 14, wherein the flow rate is in the range of 2mL/min and 4mL/min.

## Patentansprüche

1. Verneblervorrichtung (1), umfassend einen in einem Gehäuse (2) installierten Aerosolerzeuger (10), eine Steuereinheit (11) und ein Mundstück (6, 3), wobei die Vorrichtung zum Zuführen eines Wirkstoffs in einem Aerosol in die Mundhöhle angepasst ist, wobei:
der Aerosolerzeuger eine Lochplatte (15) und einen Vibrationsaktor (11, 17) zum Vibrieren der Lochplatte mit einer Frequenz zur Aerosolierung einer Flüssigkeit umfasst, wobei die Lochplatte Löcher mit einem Austrittsdurchmesser von mehr als 5,0 µm aufweist;
die Vorrichtung ein Lenkmittel (3, 6) zum Lenken eines Exhalationsstroms in Richtung des Aerosolerzeugers (10) zum Mitreißen von Aerosol umfasst;
das Mundstück Merkmale (21) zum Verstärken von turbulenter Strömung von Luft mit mitgerissenem Aerosol umfasst, wobei die Merkmale Oberflächengebilde (21) in inneren Oberflächen einer einen Strömungsweg in dem Mundstück bereitstellenden Strömungsleitung (6, 25) umfassen und den Strömungsweg verengen;
die Vorrichtung einen Detektor (12) zum Erkennen von Inhalation und/oder Exhalation umfasst; und
die Steuereinheit (11) dazu konfiguriert ist, derart zu arbeiten, dass der Betrieb des Aerosolerzeugers (10) auf das Ende einer Ateminhalation reagiert.

2. Vorrichtung nach Anspruch 1, wobei der Aerosolerzeuger (10) dazu angepasst ist, Tröpfchen bereitzustellen, von denen eine Großteilsfraktion eine Größe von mehr als 10 µm aufweist.

3. Vorrichtung nach Ansprüchen 1 oder 2, wobei die Lochplatte Löcher mit einem Austrittsdurchmesser von mehr als 6,8 µm aufweist.

4. Vorrichtung nach Ansprüchen 2 oder 3, wobei die Großteilsfraktion 75 % und vorzugsweise 90 % beträgt.

5. Vorrichtung nach einem der vorangehenden Ansprüche, wobei die Merkmale Höcker oder Wulste (21) umfassen.

6. Vorrichtung nach einem der vorangehenden Ansprüche, wobei die Steuereinheit zum Betrieb des Aerosolerzeugers (10) als Reaktion auf nasale Exhalation konfiguriert ist.

7. Vorrichtung nach einem der vorangehenden Ansprüche, wobei der Detektor eines oder mehrere von Temperatur-, Druck-, Strömungs- oder akustischen Sensoren umfasst.

8. Vorrichtung nach Anspruch 7, wobei der Sensor einen Drucksensor (12) umfasst und der Drucksensor (12) stromaufwärts des Aerosolerzeugers (10) an oder benachbart zu einem geschlossenen Ende einer Kammer (25) proximal eines Auslasses (3) des Mundstücks installiert ist.

9. Vorrichtung nach Anspruch 8, wobei das Mundstück (6) in der Reihenfolge von proximal nach distal in Richtung des Munds des Benutzers im Gebrauch Folgendes umfasst: einen Strömungs- oder Drucksensor (12), den Aerosolerzeuger (10), einen von einer Wand des Gehäuses (2, 6) gebildeten Raum (25) mit Merkmalen (21), die den Strömungsweg verengen, und ein Auslassgitter (3).

10. Vorrichtung nach einem der vorangehenden Ansprüche, wobei die Steuereinheit dazu konfiguriert ist, den folgende Schritte umfassenden Betrieb der Vorrichtung zu steuern:
Aerosolieren einer einen Wirkstoff enthaltenden Flüssigkeit, um ein Tröpfchen mit einem durchschnittlichen mittleren Durchmesser von mehr als 10 µm umfassendes Aerosol bereitzustellen;
Starten der Aerosolerzeugung bei oder nach dem Ende der Ateminhalation;
Zuführen des Aerosols in die Mundhöhle; und
Beenden der Aerosolerzeugung bei oder vor dem Beginn der Ateminhalation.

11. Vorrichtung nach einem der vorangehenden Ansprüche, wobei die Steuereinheit (11) dazu angepasst ist, die Treiberspannung für die Lochplatte (15) zu verändern, um eine gewünschte Tröpfchengröße zu erreichen.

12. Vorrichtung nach einem der vorangehenden Ansprüche, wobei der Aerosolerzeuger eine Lochplatte mit Aerosolbildungslöchern umfasst und die Lochplatte eine Speicherschicht mit Hohlräumen, die Flüssigkeitszufuhröffnungen für die Aersosolbildungslöcher bilden, umfasst.

13. Vorrichtung nach einem der vorangehenden Ansprüche, wobei der Aerosolerzeuger eine Lochplatte umfasst, die eine Anzahl von Aerosolbildungslöchern von mehr als 200 und vorzugsweise mehr als 1000 aufweist.

14. Vorrichtung nach einem der vorangehenden Ansprüche, wobei die Steuereinheit (11) und der Aerosolerzeuger (10) dazu konfiguriert sind, Volumenströme von Aerosol von mehr als 1 ml/min und unter 5 ml/min bereitzustellen.

15. Vorrichtung nach Anspruch 14, wobei der Volumenstrom im Bereich von 2 ml/min und 4 ml/min liegt.

## Revendications

1. Dispositif formant nébuliseur (1) comportant un générateur d'aérosol (10) monté dans un boîtier (2), un dispositif de commande (11), et un embout buccal (6, 3), le dispositif étant adapté à des fins d'administration d'un agent actif dans un aérosol jusque dans la cavité buccale,
dans lequel :
le générateur d'aérosol comporte une plaque à orifices (15) et un actionneur à vibrations (11, 17) servant à faire vibrer la plaque à orifices à une fréquence à des fins d'aérosolisation d'un liquide, dans lequel la plaque à orifices a des orifices ayant un diamètre de sortie dépassant 5,0 µm ;
le dispositif comporte un moyen de direction (3, 6) servant à diriger un écoulement d'expiration vers le générateur d'aérosol (10) à des fins d'entraînement de l'aérosol ;
l'embout buccal comporte des particularités (21) servant à améliorer l'écoulement d'air turbulent avec l'aérosol entraîné, lesdites particularités comportant des formations de surface (21) dans des surfaces internes d'un conduit d'écoulement (6, 25) à des fins de mise en oeuvre d'un trajet d'écoulement dans l'embout buccal, et à des fins de rétrécissement dudit trajet d'écoulement ;
le dispositif comporte un détecteur (12) servant à détecter l'inspiration et/ou l'expiration ; et
le dispositif de commande (11) est configuré pour fonctionner de telle sorte que le fonctionnement du générateur d'aérosol (10) est réactif par rapport à la fin d'une inspiration de la respiration.

2. Dispositif selon la revendication 1, dans lequel le générateur d'aérosol (10) est adapté pour fournir des gouttelettes dont une fraction majoritaire a une taille supérieure à 10 µm.

3. Dispositif selon les revendications 1 ou 2, dans lequel la plaque à orifices a des orifices ayant un diamètre de sortie dépassant 6,8 µm.

4. Dispositif selon les revendications 2 ou 3, dans lequel la fraction majoritaire est de 75 %, et de préférence de 90 %.

5. Dispositif selon l'une quelconque des revendications précédentes, dans lequel les particularités comportent des dépressions ou des nervures (21).

6. Dispositif selon l'une quelconque des revendications précédentes, dans lequel le dispositif de commande est configuré à des fins de fonctionnement du générateur d'aérosol (10) en réaction à une expiration par le nez.

7. Dispositif selon l'une quelconque des revendications précédentes, dans lequel le détecteur comporte un ou plusieurs parmi des capteurs de température, de pression, d'écoulement ou de son.

8. Dispositif selon la revendication 7, dans lequel le capteur comporte un capteur de pression (12), et le capteur de pression (12) est monté en amont du générateur d'aérosol (10) au niveau d'une, ou de manière adjacente par rapport à une, extrémité fermée d'une chambre (25) de manière proximale par rapport à une sortie (3) de l'embout buccal.

9. Dispositif selon la revendication 8, dans lequel l'embout buccal (6) comporte, dans l'ordre allant de proximal à distal vers la bouche de l'utilisateur lors de l'utilisation : un capteur d'écoulement ou de pression (12), le générateur d'aérosol (10), un volume (25) formé par une paroi du boîtier (2, 6) avec des particularités (21) qui rétrécissent le trajet d'écoulement, et un crible de sortie (3).

10. Dispositif selon l'une quelconque des revendications précédentes, dans lequel le dispositif de commande est configuré pour commander le fonctionnement du dispositif y compris les étapes consistant à :
effectuer une aérosolisation d'un liquide contenant une substance active pour fournir un aérosol comportant des gouttelettes ayant un diamètre moyen de plus de 10 µm ;
commencer la génération d'aérosol au moment ou après la fin d'une inspiration de la respiration ;
administrer l'aérosol jusque dans la cavité buccale ; et
arrêter la génération d'aérosol au moment ou avant le début d'une inspiration de la respiration.

11. Dispositif selon l'une quelconque des revendications précédentes, dans lequel le dispositif de commande (11) est adapté pour faire varier la tension d'entraînement pour la plaque à orifices (15) afin d'obtenir une taille souhaitée des gouttelettes.

12. Dispositif selon l'une quelconque des revendications précédentes, dans lequel le générateur d'aérosol comporte une plaque à orifices dotée d'orifices de formation d'aérosol, et la plaque à orifices comporte une couche formant réservoir avec des cavités pour former des ouvertures d'alimentation en liquide pour les orifices de formation d'aérosol.

13. Dispositif selon l'une quelconque des revendications précédentes, dans lequel le générateur d'aérosol comporte une plaque à orifices qui a un nombre d'orifices de formation d'aérosol dépassant 200, et de préférence dépassant 1000.

14. Dispositif selon l'une quelconque des revendications précédentes, dans lequel le dispositif de commande (11) et le générateur d'aérosol (10) sont configurés pour fournir des débits d'écoulement d'aérosol dépassant 1 mL/min et inférieurs à 5 mL/min.

15. Dispositif selon la revendication 14, dans lequel le débit d'écoulement se trouve dans la plage de 2 mL/min et 4 mL/min.
